(19) Europäisches Patentamt
European Patent Office
Office européen des brevets

(11) **EP 4 772 112 A1**

(12) **EUROPEAN PATENT APPLICATION**
published in accordance with Art. 153(4) EPC

(43) Date of publication:
**08.07.2026 Bulletin 2026/28**

(21) Application number: **24859997.9**

(22) Date of filing: **30.08.2024**

(51) International Patent Classification (IPC):
***A61B 5/055*** (2006.01)

(52) Cooperative Patent Classification (CPC):
**A61B 5/055**

(86) International application number:
**PCT/JP2024/031370**

(87) International publication number:
**WO 2025/047972 (06.03.2025 Gazette 2025/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.08.2023 JP 2023139863**

(71) Applicant: **National University Corporation Chiba
University**
**Chiba-shi, Chiba 263-8522 (JP)**

(72) Inventors:
• **EGUCHI, Yawara**
**Chiba-shi, Chiba 260-8670 (JP)**
• **ORITA, Sumihisa**
**Chiba-shi, Chiba 263-8522 (JP)**
• **TAKEUCHI, Hidenari**
**Chiba-shi, Chiba 263-8522 (JP)**
• **MASUMOTO, Rira**
**Chiba-shi, Chiba 263-8522 (JP)**

(74) Representative: **Hoffmann Eitle
Patent- und Rechtsanwälte PartmbB
Arabellastraße 30
81925 München (DE)**

(54) **MEDICAL IMAGE PROCESSING DEVICE**

(57)    An object of the present invention is to provide a medical image processing device for automating measurement of FA values. Provided as a solution is: a medical image processing device comprising a diffusion tensor imaging MRI data acquisition unit that acquires diffusion tensor imaging MRI data obtained by scanning the lumbar region of a patient with low back and leg pain, a coordinate information estimation unit that estimates coordinate information of a nerve root in a designated axial cross-section, and a measurement unit that measures an FA value of a nerve root of which coordinate information was estimated.

[Fig. 4]

EP 4 772 112 A1

**Description**

Technical Field

The present invention relates to a medical image processing device, a method for acquiring information on a spinal nerve, a method for generating diffusion tensor tractography indicating tracts of nerve fibers of the spinal nerve, and a method and a program for generating a 3D FA map.

Background Art

Low back pain is the number one complaint among both men and women in Japan.

The Clinical Practice Guidelines on the Management of Low Back Pain (2019) state that magnetic resonance imaging (MRI) diagnosis is essential for diagnosing low back pain. While lumbar nerve disorders cause low back and leg pain, asymptomatic intervertebral disk degeneration and herniation are frequently observed, and conventional MRI diagnosis often fails to establish that nerve root compression being visible in an image is necessarily the cause of pain.

Therefore, functional assessment including visualization of damaged nerves and quantification of pain was difficult.

Diffusion tensor imaging (DTI) that uses MRI to enhance a directionality of water molecule diffusion within biological tissue enables visualization of nerve fibers in a non-invasive manner by tracking diffusion anisotropy of the nerve fibers.

In particular, it has been reported that DTI enables three-dimensional tracts of the lumbar plexus to be clearly visualized. Furthermore, it has been reported that when a disruption in tractography is observed in a part of lumbar foraminal stenosis of which diagnosis is considered difficult, a fractional anisotropy (FA) value that is an indicator of diffusion anisotropy strength decreases and correlates with clinical symptoms (NPL 1 to 3).

Citation List

Non Patent Literature

NPL 1: Eguchi Y, Ohtori S, Orita S, et al., Preliminary Results. American Journal of Neuroradiology 2011; 32: 1824-1829.
NPL 2: Eguchi Y, Ohtori S, Yamashita M, et al., European Spine Journal 2010;19:1874-1882.
NPL 3: Eguchi Y, Ohtori S, Yamashita M, et al., Neuroradiology 2011; 53: 633-641.

Summary of Invention

Technical Problem

However, measuring FA values and creating tractography from DTI data are manual operations and, therefore, require time and experience, and it cannot be said that these techniques are widely adopted in clinical practice.

In order to solve the problem described above, an object of the present invention is to provide a medical image processing device for automating FA value measurement, a method for acquiring information on a spinal nerve, a method for generating diffusion tensor tractography indicating tracts of nerve fibers of the spinal nerve, and a method and a program for generating a 3D FA map.

Solution to Problem

The present invention encompasses the following aspects [1] to [14] and [6-1].

[1] A medical image processing device comprising: a diffusion tensor imaging MRI data acquisition unit that acquires diffusion tensor imaging MRI data obtained by scanning the lumbar region of a patient with low back and leg pain;

a coordinate information estimation unit that estimates coordinate information of a nerve root in a designated axial cross-section; and
a measurement unit that measures an FA value of a nerve root of which coordinate information was estimated, wherein

the diffusion tensor imaging MRI data includes diffusion-weighted image information and FA map information containing scalar information, vector information, and coordinate information,

the coordinate information estimation unit estimates coordinate information of the nerve root in the designated axial cross-section using a pre-trained model trained using, as training data, annotated images in which annotations were added to two nerve root regions and a spinal canal region included in a same axial cross-sectional image among MRI diffusion-weighted images of the lumbar region, and

the measurement unit measures, based on coordinate information of the nerve root estimated by the coordinate information estimation unit, an FA value of the nerve root of which coordinate information was estimated based on the scalar information and the coordinate information.

[2] The medical image processing device according to [1], wherein the same axial cross-sectional image among the MRI diffusion-weighted images of the lumbar region is a cross-sectional image of at least one of the third lumbar vertebra (L3), the fourth lumbar vertebra (L4), and the fifth lumbar vertebra (L5).

[3] The medical image processing device according to [2], wherein the same axial cross-sectional image among the MRI diffusion-weighted images of the lumbar region is a cross-sectional image of the third lumbar vertebra (L3), the fourth lumbar vertebra (L4), and the fifth lumbar vertebra (L5).

[4] The medical image processing device according to any one of [1] to [3], wherein the same axial cross-sectional image among the MRI diffusion-weighted images of the lumbar region is a plurality of cross-sectional images centered on a central portion of a same nerve root.

[5] The medical image processing device according to [4], wherein the same axial cross-sectional image among the MRI diffusion-weighted images of the lumbar region is a cross-sectional image of proximal, central, and distal portions of a same nerve root.

[6] The medical image processing device according to any one of [1] to [5], wherein in the training data, the annotation of the nerve root region differs from the annotation of the spinal canal region.

[6-1] The medical image processing device according to any one of [1] to [5], wherein the coordinate information estimation unit estimates coordinates of the nerve root after removing only the spinal canal region through image processing.

[7] The medical image processing device according to any one of [1] to [6] and [6-1], wherein the designated axial cross-section is a plurality of consecutive axial cross-sections in the lumbar region of the patient, and the medical image processing device further comprises:

a tractography generation unit that associates coordinate information of the nerve root in each axial cross-section estimated by the coordinate information estimation unit with the scalar information, the vector information, and the coordinate information of the FA map information created from the diffusion tensor imaging MRI data and that generates diffusion tensor tractography indicating a tract of nerve fibers.

[8] The medical image processing device according to any one of [1] to [6] and [6-1], wherein the designated axial cross-section is a plurality of consecutive axial cross-sections in the lumbar region of the patient, and the medical image processing device further comprises:

a 3D FA map generation unit that associates coordinate information of the nerve root in each axial cross-section estimated by the coordinate information estimation unit with an FA value measured by the measurement unit, reconstructs the nerve root in a three-dimensional space, and generates a 3D FA map.

[9] A method for acquiring information on a spinal nerve, comprising the steps of:

acquiring diffusion tensor imaging MRI data obtained by scanning the lumbar region of a patient with low back and leg pain;

estimating coordinate information of a nerve root in a designated axial cross-section; and

measuring an FA value of a nerve root of which coordinate information was estimated, wherein

the diffusion tensor imaging MRI data includes diffusion-weighted image information and FA map information containing scalar information, vector information, and coordinate information,

the step of estimating the coordinate information involves estimating coordinate information of the nerve root in the designated axial cross-section using a pre-trained model trained using, as training data, annotated images in which annotations were added to two nerve root regions and a spinal canal region included in a same axial cross-sectional image among MRI diffusion-weighted images of the lumbar region, and

the step of measuring an FA value of the nerve root involves measuring, based on coordinate information of the nerve root estimated in the step of estimating coordinate information, an FA value of the nerve root of which coordinate information was estimated based on the scalar information and the coordinate information.

[10] The method according to [9], wherein information on a spinal nerve acquired by the method according to [9] is an index for estimating the degree of spinal nerve disorder from a measured FA value.

[11] A method for generating diffusion tensor tractography indicating a tract of nerve fibers of a spinal nerve, the method comprising the steps of:

acquiring diffusion tensor imaging MRI data obtained by scanning the lumbar region of a patient with low back and leg pain;

estimating coordinate information of a nerve root in each of a plurality of consecutive axial cross-sections in a lumbar region of a designated patient;

associating the information; and

generating diffusion tensor tractography indicating a tract of nerve fibers by superimposing each axial cross-section to three-dimensionally construct the axial cross-sections, wherein

the diffusion tensor imaging MRI data includes diffusion-weighted image information and FA map information containing scalar information, vector information, and coordinate information,

the step of estimating the coordinate information involves estimating coordinate information of the nerve root in the designated axial cross-section using a pre-trained model trained using, as training data, annotated images in which annotations were added to two nerve root regions and a spinal canal region included in a same axial cross-sectional image among MRI diffusion-weighted images of the lumbar region, and

the step of associating the information involves associating coordinate information of the nerve root in each axial cross-section estimated in the step of estimating the coordinate information of the nerve root with the scalar information, the vector information, and the coordinate information of the FA value map created from the diffusion tensor imaging MRI data.

[12] A method for generating a 3D FA map, comprising the steps of:

acquiring diffusion tensor imaging MRI data obtained by scanning the lumbar region of a patient with low back and leg pain;

estimating coordinate information of a nerve root in each of a plurality of consecutive axial cross-sections in a lumbar region of a designated patient;

measuring an FA value of a nerve root of which coordinate information was estimated; and

reconstructing the nerve root in a three-dimensional space using coordinate information of the nerve root of which coordinate information was estimated and the measured FA value corresponding to the coordinate information, wherein

the diffusion tensor imaging MRI data includes diffusion-weighted image information and FA map information containing scalar information, vector information, and coordinate information,

the step of estimating the coordinate information involves estimating coordinate information of the nerve root in the designated axial cross-section using a pre-trained model trained using, as training data, annotated images in which annotations were added to two nerve root regions and a spinal canal region included in a same axial cross-sectional image among MRI diffusion-weighted images of the lumbar region, and

the step of measuring an FA value of the nerve root involves measuring, based on coordinate information of the nerve root estimated in the step of estimating coordinate information, an FA value of the nerve root of which coordinate information was estimated based on the scalar information and the coordinate information.

[13] The method for generating a 3D FA map according to [12], further comprising the steps of: comparing measured FA values of two nerve roots included in a same axial cross-sectional image and of which coordinate information was estimated; and

distinguishably displaying a region of nerve roots where a predetermined difference exists between the measured FA values of the two nerve roots included in a same axial cross-sectional image and of which coordinate information was estimated from a region of other nerve roots.

[14] A program for causing a computer to execute each step of the method for acquiring information on a spinal nerve according to [9] or [10], the method for generating diffusion tensor tractography indicating tracts of nerve fibers of a spinal nerve according to [11], or the method for generating a 3D FA map according to [12] or [13].

Advantageous Effects of Invention

The present invention provides a medical image processing device for automating FA value measurement, a method for acquiring information on a spinal nerve, a method for generating diffusion tensor tractography indicating tracts of nerve fibers of the spinal nerve, and a method and a program for generating a 3D FA map. Furthermore, in a preferable aspect of the present invention, automation of tractography creation is also possible.

Brief Description of Drawings

[Fig. 1] Fig. 1 is a diagram showing parameters representing anisotropic diffusion in coordinates (X, Y, Z). This indicates that the direction of motion of water molecules is restricted, resulting in anisotropic diffusion.

[Fig. 2] Fig. 2A is a diagram showing an annotation target region. Fig. 2B is a diagram showing an example where annotation is performed in two nerve root regions and a spinal canal region.

[Fig. 3] Fig. 3 is a diagram showing a structure of U-Net.

[Fig. 4] Fig. 4 is a diagram showing an original image (left) and an actual predicted image (right) obtained during evaluation of a trained model.

[Fig. 5] Fig. 5 shows a diffusion-weighted image and an annotated image used as training data in the trained model of [1-1].

[Fig. 6] Fig. 6 shows a result produced by the trained model of [1-1] and an extracted image.

[Fig. 7] Fig. 7 is a diagram schematically showing a method for measuring FA values in an automated manner.

[Fig. 8] Fig. 8 is a diagram showing a comparison result between automated measurement (AI measurement) of FA values and manual measurement of FA values.

[Fig. 9] Fig. 9 shows an example where locations of a disruption match between tractography and a 3D FA map.

Description of Embodiments

[Medical image processing device]

A medical image processing device according to the present invention is a medical image processing device comprising: a diffusion tensor imaging MRI data acquisition unit that acquires diffusion tensor imaging MRI data obtained by scanning the lumbar region of a patient with low back and leg pain;

a coordinate information estimation unit that estimates coordinate information of a nerve root in a designated axial cross-section; and

a measurement unit that measures an FA value of a nerve root of which coordinate information was estimated, wherein

the diffusion tensor imaging MRI data includes diffusion-weighted image information and FA map information containing scalar information, vector information, and coordinate information,

the coordinate information estimation unit estimates coordinate information of the nerve root in the designated axial cross-section using a pre-trained model trained using, as training data, annotated images in which annotations were added to two nerve root regions and a spinal canal region included in a same axial cross-sectional image among MRI diffusion-weighted images of the lumbar region, and

the measurement unit measures, based on coordinate information of the nerve root estimated by the coordinate information estimation unit, an FA value of the nerve root of which coordinate information was estimated based on the scalar information and the coordinate information.

< Patient >

The medical image processing device according to the present invention is intended for patients with low back and leg pain.

Low back and leg pain refers to either or both low back pain and leg pain. Patients with low back and leg pain include, for example, those who experience subjective symptoms of low back and leg pain.

Low back and leg pain is primarily caused by lumbar nerve compression, lumbar foraminal stenosis, and the like at one or more of the third lumbar vertebra (L3), the fourth lumbar vertebra (L4), and the fifth lumbar vertebra (L5) in the lumbar region. One of the objects of the present invention is to provide a medical image processing device for achieving visualization and quantitative evaluation of low back and leg pain caused by nerve disorders associated with lumbar nerve compression, lumbar foraminal stenosis, and the like. Visualization and quantitative evaluation are expected to contribute to auxiliary diagnosis.

< Anisotropic diffusion >

Hereinafter, anisotropic diffusion will be described in detail.

In nerve fibers, the axonal cell membrane and the myelin sheath impede diffusion of water in a direction that heads orthogonalto the nerve fiber bundle. Consequently, the nerve fibers have lost isotropic diffusion (isotropy) of water molecules and exhibit anisotropic diffusion (anisotropy) instead.

An FA (Fractional Anisotropy) value is an index that indicates strength of anisotropic diffusion. The FA value ranges from 0 to 1, with values closer to 1 indicating a state with stronger anisotropic diffusion, while 0 indicates completely isotropic diffusion.

In nerve fibers, it is known that nerve damage causes a decrease in diffusion anisotropy along the nerve fibers, resulting in a decrease in the FA value. Therefore, the FA value is known to enable quantitative evaluation of nerve damage. Accordingly, the FA value is suitably used as an indicator of the degree of spinal nerve disorder.

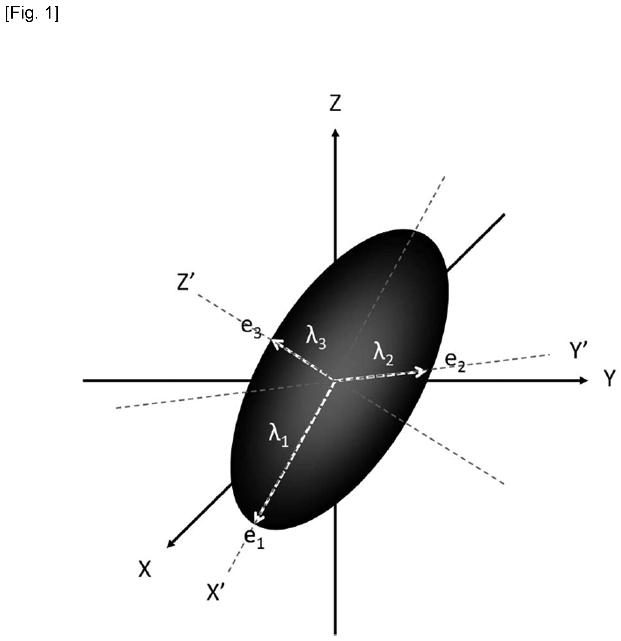

Fig. 1 shows parameters representing anisotropic diffusion in coordinates (X, Y, Z). The respective axes of the coordinates (X, Y, Z) correspond to a coordinate system of MRI data. Fig. 1 indicates that the direction of motion of water molecules is restricted and that anisotropic diffusion has occurred.

As shown in the drawing, a direction exhibiting strongest diffusion is defined as X' while the two directions perpendicular to X' are defined as Y' and Z', and diffusion coefficients for the respective directions are represented by $\lambda_1 \lambda_2$, and $\lambda_3$ (where $\lambda_1 \geq \lambda_2 \geq \lambda_3$). $\lambda_1$, $\lambda_2$, and $\lambda_3$ are referred to as eigenvalues. Furthermore, vectors of the respective directions corresponding to the respective eigenvalues ($\lambda_1 \lambda_2$, and $\lambda_3$) are represented by $e_1$, $e_2$, and $e_3$ and are referred to as eigenvectors.

In the present specification, combinations of the eigenvalues ($\lambda_1 \lambda_2$, and $\lambda_3$) and the eigenvectors ($e_1$, $e_2$, and $e_3$) are referred to as vector information. Once vector information is obtained, the degree of anisotropic diffusion within voxels can be quantitatively calculated.

The eigenvalues ($\lambda_1 \lambda_2$, and $\lambda_3$) and the eigenvectors ($e_1$, $e_2$, and $e_3$) enable anisotropic diffusion at specific coordinates (X, Y, Z) to be quantitatively expressed.

A diffusion tensor D at coordinates (X, Y, Z) is given by a 3 × 3 determinant shown in Equation (Eq. 1). Here, for example, $D_{XX}$ represents a diffusion coefficient when a motion detection magnetic field gradient (Motion Problem Gradient: MPG) is applied in the X-axis direction. By obtaining diffusion coefficients ($D_{XX}$, $D_{XY}$, $D_{XZ}$, Dyy, $D_{YZ}$, and $D_{ZZ}$) with respect to the six directions of XX, XY, XZ, YY, YZ, and ZZ, respectively, and diagonalize the diffusion tensor D shown in Equation (Eq. 1), the respective eigenvalues ($\lambda_1 \lambda_2$, and $\lambda_3$) can be calculated as shown in Equation (Eq. 2).

[Expression 1]

$$D = \begin{pmatrix} D_{XX} & D_{XY} & D_{XZ} \\ D_{XY} & D_{YY} & D_{ZY} \\ D_{XZ} & D_{YZ} & D_{ZZ} \end{pmatrix} \qquad (\text{Eq. 1})$$

$$D = \begin{pmatrix} \lambda_1 & 0 & 0 \\ 0 & \lambda_2 & 0 \\ 0 & 0 & \lambda_3 \end{pmatrix} \qquad (\text{Eq. 2})$$

An apparent diffusion coefficient (ADC) can be calculated from the respective obtained eigenvalues ($\lambda_1 \lambda_2$, and $\lambda_3$) using Equation (Eq. 3) and, furthermore, a FA value (fractional anisotropy value) can be calculated using Equation (Eq. 4).

[Expression 2]

$$ADC = \frac{\lambda_1 + \lambda_2 + \lambda_3}{3} = D_{ave} \qquad (\text{Eq. 3})$$

$$FA = \sqrt{\frac{3}{2} \cdot \frac{\left(\lambda_1 - D_{ave}\right)^2 + \left(\lambda_2 - D_{ave}\right)^2 + \left(\lambda_3 - D_{ave}\right)^2}{\lambda_1^2 + \lambda_2^2 + \lambda_3^2}}$$

$$(\text{Eq. 4})$$

< Diffusion tensor imaging MRI data acquisition unit >

The medical image processing device according to the present invention is equipped with a diffusion tensor imaging MRI data acquisition unit.

In the present specification, "MRI" means magnetic resonance imaging. MRI measurements are taken in voxel units, and the coordinates described earlier typically refer to voxel coordinates.

The diffusion tensor imaging MRI data acquisition unit acquires diffusion tensor imaging MRI data scanned by an MRI device. The diffusion tensor imaging MRI data may be data scanned immediately prior or data scanned in the past. In case of data scanned in the past, for example, data stored in a computer-readable storage medium can be used. The diffusion tensor imaging MRI data can be scanned or, for example, captured using known MRI devices.

The diffusion tensor imaging MRI data is data obtained by scanning the lumbar region of a patient with low back and leg pain. The lumbar region of a patient refers to a region including the spinal canal and its surroundings, and more preferably includes the spinal canal and surroundings of the third lumbar vertebra (L3), the fourth lumbar vertebra (L4), and the fifth lumbar vertebra (L5).

(Diffusion tensor imaging MRI data)

The diffusion tensor imaging MRI data includes diffusion-weighted image information and FA map information.

· Diffusion-weighted image information

A diffusion-weighted image (DWI) emphasizes and visualizes a movement (diffusion) of water molecules and, for example, the image is created by applying a motion detection magnetic field gradient (Motion Problem Gradient: MPG) from a certain direction to a diffusion motion of protons.

· FA map information

FA map information includes scalar information, vector information, and coordinate information.

In other words, FA map information associates each coordinate (X, Y, Z) with scalar information indicating a magnitude of the FA value and a combination of the eigenvalues ($\lambda_1$ $\lambda_2$, and $\lambda_3$) and the eigenvectors ($e_1$, $e_2$, and $e_3$) described earlier.

< Coordinate information estimation unit >

The medical image processing device according to the present invention is equipped with a coordinate information estimation unit that estimates coordinate information of a nerve root in a designated axial cross-section in the diffusion-weighted image.

An axial cross-section refers to a cross-section that heads orthogonal to a body axis being a line connecting a head region and a tail region of the subject being measured.

The axial cross-section is designated by, for example, a measured subject designation unit that the medical image processing device according to the present invention is further equipped with. The axial cross-section can be designated by, for example, an operator of the medical image processing device via the measured subject designation unit. Alternatively, the medical image processing device may be configured to automatically designate the axial cross-section.

The coordinate information estimation unit estimates coordinate information of the nerve root in the designated axial cross-section using a pre-trained model trained using, as training data, annotated images in which annotations were added to typically two nerve root regions and typically one spinal canal region included in a same axial cross-sectional image among MRI diffusion-weighted images of the lumbar region.

The pre-trained model has an algorithm that causes an artificial neural network structured from many layers of neurons to perform computations to extract patterns from teaching data and to apply those patterns to new data to output estimation results.

In one preferable aspect, the coordinate information estimation unit estimates coordinates of a nerve root after removing only the spinal canal region through image processing. Specifically, since the spinal canal region represents the largest area in the image, removing the largest area allows for the removal of only the spinal canal region. This is particularly suitable when annotation of the nerve root region and annotation of the spinal canal region are the same in training data (to be described later).

(Training data)

Hereinafter, training data and preferable aspects thereof will be described in detail.

The pre-trained model used by the coordinate information estimation unit is trained using, as training data, annotated images in which annotations were added to typically two nerve root regions and typically one spinal canal region included in a same axial cross-sectional image among MRI diffusion-weighted images of the lumbar region.

Using annotated images in which annotations were added to two nerve root regions and one spinal canal region improves accuracy of estimated coordinate information of the nerve root. The annotation of the nerve root region and the annotation of the spinal canal region may be the same or may differ from each other.

In one preferable aspect, the annotation of the nerve root region and the annotation of the spinal canal region differ from each other. In another preferable aspect, the annotation of the nerve root region and the annotation of the spinal canal region are the same.

The MRI diffusion-weighted images of the lumbar region to be used in training data can be acquired from both healthy individuals and patients. From the perspective of data standardization, the MRI diffusion-weighted images are preferably acquired from healthy individuals.

The axial cross-sectional image is an axial cross-sectional image of at least one of the third lumbar vertebra (L3), the fourth lumbar vertebra (L4), and the fifth lumbar vertebra (L5) and, more preferably, three axial cross-sectional images of the third lumbar vertebra (L3), the fourth lumbar vertebra (L4), and the fifth lumbar vertebra (L5).

In addition, the MRI diffusion-weighted image of the lumbar region used in training data is preferably a plurality of cross-sectional images centered on a central portion of a same nerve root. In particular, the MRI diffusion-weighted image is more preferably cross-sectional images of the proximal, central, and distal portions of the same nerve root. In healthy nerves, the FA value tends to increase proximally, centrally, and distally along the nerve root. FA values between the unaffected and compressed sides are desirably compared at a plurality of locations centered on the central portion of the same nerve root within a same slice to compare relative changes.

< Measurement unit >

The medical image processing device according to the present invention is equipped with a measurement unit that measures, based on coordinate information of the nerve root estimated by the coordinate information estimation unit, an FA value of the nerve root of which coordinate information was estimated based on the scalar information and the coordinate information.

Specifically, scalar information associated with coordinate information in the FA map information corresponding to the coordinate information of the nerve root of which the coordinate information was estimated is specified as the FA value of the nerve root of which the coordinate information was estimated. The estimated coordinate information of the nerve root typically includes a plurality of voxels. In such a case, an average value of the FA values of the respective voxels is specified as the FA value of the nerve root of which the coordinate information was estimated.

< Diffusion tensor tractography >

Diffusion tensor tractography (DTT: hereinafter, simply referred to as tractography) refers to imaging technique that enables three-dimensional tracking and visualization of anisotropic nerve fibers based on a diffusion tensor. Tractography enables visualization of disruptions in nerve tracts.

Tractography sets a starting point (seed) for any fiber tracking within any region set as a region of interest (ROI), and calculates the eigenvalues ($\lambda_1$, $\lambda_2$, $\lambda_3$) and the eigenvectors ($e_1$, $e_2$, $e_3$) for the starting point. The eigenvalues ($\lambda_1$, $\lambda_2$, $\lambda_3$) and the eigenvectors ($e_1$, $e_2$, $e_3$) of next coordinates encountered along the vector $e_1$ that is a direction of maximum diffusion are determined. The same process is further repeated along the vector $e_1$ that is a direction of maximum diffusion at the coordinates. A tract of nerve fibers can be tracked in this manner, and this method is also referred to as fiber tracking.

< Tractography generation unit >

The medical image processing device according to the present invention can also be equipped with a tractography generation unit.

The tractography generation unit associates coordinate information of the nerve root in each axial cross-section estimated by the coordinate information estimation unit in a plurality of consecutive axial cross-sections with the scalar information, the vector information, and the coordinate information, and generates diffusion tensor tractography indicating a tract of nerve fibers.

Specifically, for example, diffusion tensor tractography is generated as follows. First, in one axial cross-section selected from a plurality of consecutive axial cross-sections, an ROI to be a starting point for fiber tracking based on the coordinate information of the nerve root of which the coordinate information has been estimated. Then, from the vector information associated with the coordinate information of the nerve root, the eigenvalues ($\lambda_1$, $\lambda_2$, $\lambda_3$) and the eigenvectors ($e_1$, $e_2$, $e_3$) in that ROI are determined. Accordingly, a direction of a diffusion tensor in the ROI is determined. Next, tracking is started along a direction $e_1$ where diffusion is greatest. Similarly, the diffusion tensor (in other words, each eigenvalue and each eigenvector) is calculated for a voxel corresponding to the coordinate information of the nerve root encountered in the travel direction $e_1$. Each of the eigenvalues includes the eigenvalues ($\lambda_1$, $\lambda_2$, $\lambda_3$) and a FA value calculated from the eigenvalues ($\lambda_1$, $\lambda_2$, $\lambda_3$). The same process is repeated along the vector $e_1$ that is a direction of maximum diffusion in the plurality of consecutive axial cross-sections to perform fiber tracking in succession to obtain three-dimensionally constructed tractography. On the other hand, the fiber tracking is terminated when encountering a non-continuous voxel.

As another aspect, diffusion tensor tractography can be generated, without utilizing the estimated coordinate information of the nerve root, by preparing fiber tracking data using the eigenvectors ($e_1$, $e_2$, $e_3$), the eigenvalues ($\lambda_1$, $\lambda_2$, $\lambda_3$), and the FA values calculated from the eigenvalues ($\lambda_1$, $\lambda_2$, $\lambda_3$) and, subsequently, excluding the fiber tracking data for nerves passing through coordinates other than the estimated coordinate information of the nerve root in each axial cross-section where the coordinate information was estimated.

< 3D FA map generation unit >

The medical image processing device according to the present invention can also be equipped with a 3D FA map generation unit.

When the medical image processing device is equipped with the 3D FA map generation unit, the designated axial cross-section is a plurality of consecutive axial cross-sections in the lumbar region of the patient, and the 3D FA map generation unit associates coordinate information of the nerve root in each axial cross-section estimated by the coordinate information estimation unit in the plurality of consecutive axial cross-sections with an FA value measured by the measurement unit, reconstructs the nerve root in a three-dimensional space, and generates a 3D FA map.

Preferably, the 3D FA map generation unit compares measured FA values of two nerve roots included in a same axial cross-sectional image and of which coordinate information was estimated, and distinguishably displays nerve roots in which a predetermined difference exists between the measured FA values of two nerve roots included in a same axial cross-sectional image and of which coordinate information was estimated from other nerve roots with a difference less than the predetermined difference.

The predetermined difference is, for example, a difference equal to or more than 0.1 between FA values.

Here, distinguishably displaying nerve roots in which a predetermined difference exists between the measured FA values of two nerve roots of which coordinate information was estimated from other nerve roots with a difference less than the predetermined difference includes: (1) distinguishably displaying the presence of nerve roots with a predetermined difference by only displaying such nerve roots; (2) distinguishably displaying the absence of nerve roots with a predetermined difference by only displaying nerve roots with a difference less than the predetermined difference; (3) distinguishably displaying nerve roots with the predetermined difference from nerve roots with a difference less than the predetermined difference by, for example, displaying the nerve roots in different colors or displaying the nerve roots with varying shades of color.

The medical image processing device according to the present invention is realized by a computer equipped with, for example, a CPU, a RAM, a ROM, a hard disk, a display, a keyboard, a mouse, a communication interface, and the like.

[Method for acquiring information on a spinal nerve]

The present invention also provides a method for acquiring information on a spinal nerve.

The method for acquiring information on a spinal nerve according to the present invention comprises the steps of:

acquiring diffusion tensor imaging MRI data obtained by scanning the lumbar region of a patient with low back and leg pain;
estimating coordinate information of a nerve root in a designated axial cross-section; and
measuring an FA value of a nerve root of which coordinate information was estimated, wherein
the diffusion tensor imaging MRI data includes diffusion-weighted image information and FA map information containing scalar information, vector information, and coordinate information,
the step of estimating the coordinate information involves estimating coordinate information of the nerve root in the designated axial cross-section using a pre-trained model trained using, as training data, annotated images in which annotations were added to two nerve root regions and a spinal canal region included in a same axial cross-sectional image among MRI diffusion-weighted images of the lumbar region, and
the step of measuring an FA value of the nerve root involves measuring, based on coordinate information of the nerve root estimated in the step of estimating coordinate information, an FA value of the nerve root of which coordinate information was estimated based on the scalar information and the coordinate information.

The information on a spinal nerve acquired by the method according to the present invention is suitably used as an index for estimating the degree of spinal nerve disorder from a measured FA value.

The method for acquiring information on a spinal nerve according to the present invention can be implemented using, for example, the medical image processing device described above.

[Method for generating diffusion tensor tractography indicating a tract of nerve fibers of a spinal nerve]

The present invention also provides a method for generating diffusion tensor tractography indicating a tract of nerve fibers of a spinal nerve.

The method for generating diffusion tensor tractography indicating a tract of nerve fibers of a spinal nerve according to the present invention comprises the steps of:

acquiring diffusion tensor imaging MRI data obtained by scanning the lumbar region of a patient with low back and leg pain;
estimating coordinate information of a nerve root in each of a plurality of consecutive axial cross-sections in a lumbar region of a designated patient;
associating the information; and
generating diffusion tensor tractography indicating a tract of nerve fibers by superimposing each axial cross-section to three-dimensionally construct the axial cross-sections, wherein
the diffusion tensor imaging MRI data includes diffusion-weighted image information and FA map information containing scalar information, vector information, and coordinate information,
the step of estimating the coordinate information involves estimating coordinate information of the nerve root in the designated axial cross-section using a pre-trained model trained using, as training data, annotated images in which annotations were added to two nerve root regions and a spinal canal region included in a same axial cross-sectional image among **MRI** diffusion-weighted images of the lumbar region, and
the step of associating the information involves associating coordinate information of the nerve root in each axial cross-section estimated in the step of estimating the coordinate information of the nerve root with the scalar information, the vector information, and the coordinate information of the FA value map created from the diffusion tensor imaging **MRI** data.

The method for generating diffusion tensor tractography indicating a tract of nerve fibers of a spinal nerve according to the present invention can be implemented using, for example, the medical image processing device described above.

[Method for generating a 3D FA map]

The present invention also provides a method for generating a 3D FA map.

A 3D FA map depicts objects containing FA value information of a nerve root along a tract of nerve fibers.

The method for generating a 3D FA map according to the present invention comprises the steps of:

acquiring diffusion tensor imaging **MRI** data obtained by scanning the lumbar region of a patient with low back and leg pain;
estimating coordinate information of a nerve root in each of a plurality of consecutive axial cross-sections in a lumbar region of a designated patient;
measuring an FA value of a nerve root of which coordinate information was estimated; and
reconstructing the nerve root in a three-dimensional space using coordinate information of the nerve root of which coordinate information was estimated and the measured FA value corresponding to the coordinate information, wherein
the diffusion tensor imaging **MRI** data includes diffusion-weighted image information and FA map information containing scalar information, vector information, and coordinate information,
the step of estimating the coordinate information involves estimating coordinate information of the nerve root in the designated axial cross-section using a pre-trained model trained using, as training data, annotated images in which annotations were added to two nerve root regions and a spinal canal region included in a same axial cross-sectional image among MRI diffusion-weighted images of the lumbar region, and
the step of measuring an FA value of the nerve root involves measuring, based on coordinate information of the nerve root estimated in the step of estimating coordinate information, an FA value of the nerve root of which coordinate information was estimated based on the scalar information and the coordinate information.

Reconstruction of the nerve root in a three-dimensional space is performed using coordinate information of the nerve root of which coordinate information was estimated and the measured FA value corresponding to the coordinate information.

The reconstruction of the nerve root in a three-dimensional space is performed by creating an object of the nerve root of which coordinate information was estimated and arranging the created object in the sequential order of the axial cross-sections. At this point, the coordinate information of the nerve root in each axial cross-section and the measured FA value corresponding to the coordinate information are associated with each other, and the created object contains FA value information of the nerve root.

Creation of the object can be performed by acquiring, for each axial cross-sectional image, contours of left and right nerve roots from the coordinate information of the nerve root of which coordinate information was estimated, and creating the object based on information on the acquired contours. The contours may be contours of the ROI of the nerve roots or rectangles circumscribing the ROI or, in other words, surrounding the nerve roots. For example, in the case of a rectangle, coordinates of the upper-left corner and vertical and horizontal lengths of the rectangle are obtained as information of a contour. The acquired information may be used to create an object after reduction. Accordingly, a 3D FA map can be displayed even when components become excessively larger during reconstruction of the nerve root.

The method for generating a 3D FA map can further comprise the steps of:

comparing measured FA values of two nerve roots included in a same axial cross-sectional image and of which coordinate information was estimated; and

distinguishably displaying a region of nerve roots where a predetermined difference exists between the measured FA values of the two nerve roots included in a same axial cross-sectional image and of which coordinate information was estimated from a region of other nerve roots.

The predetermined difference is, for example, a difference equal to or more than 0.1 between FA values. When there is the predetermined difference between FA values, nerve roots can be distinguishably displayed from other nerve roots with a difference less than the predetermined difference. Here, distinguishably displaying nerve roots in which a predetermined difference exists between the measured FA values of two nerve roots of which coordinate information was estimated from other nerve roots with a difference less than the predetermined difference includes: (1) distinguishably displaying the presence of nerve roots with a predetermined difference by only displaying such nerve roots; (2) distinguishably displaying the absence of nerve roots with a predetermined difference by only displaying nerve roots with a difference less than the predetermined difference; (3) distinguishably displaying nerve roots with the predetermined difference from nerve roots with a difference less than the predetermined difference by, for example, displaying the nerve roots in different colors or displaying the nerve roots with varying shades of color.

The generation of the 3D FA map can be performed, for example, using known software.

For example, using Three.js being software that renders 3D objects on a web browser using HTML and JavaScript files, the 3D FA map can be generated as follows.

First, a zero-padded array with a size of 40 × 40 × 40 is created using JavaScript. A csv file containing position coordinate information and FA values of nerve roots is read, and if there is a predetermined difference between the FA values, a determination is made to distinguishably display the nerve roots by, for example, differentiating the nerve roots using different colors. Using the THREE.Scene function, a 3D scene being a space for displaying objects is created, and a camera and lights are created to set a viewpoint for viewing the 3D space and positions and brightness of the lights illuminating the scene. An object is arranged in the 3D space based on the array of coordinate information of nerve roots. At this point, x, y, z coordinates of a box (object) are calculated using the getPosition function. These are added to an HTML document using the document.body.appendChild (renderer.domElement) property, and the scene as a whole is continuously redrawn on a browser by the Render function.

The 3D FA map generated by the method according to the present invention is suitably used as means for visualizing the degree of spinal nerve disorder.

The method for generating a 3D FA map according to the present invention can be implemented using, for example, the medical image processing device described above.

[Program]

The program according to the present invention is a program for causing a computer to execute each step of the method for acquiring information on a spinal nerve described above, each step of the method for generating diffusion tensor tractography indicating tracts of nerve fibers of a spinal nerve described above, or each step of the method for generating a 3D FA map described above.

Examples of the computer include a desktop or portable personal computer (PC) and a work station. The computer may be constructed by combining any number of computers of any type connected via communication networks such as the Internet or intranets.

The program is provided, recorded on a storage medium such as a CD-ROM, a DVD-ROM, and a semiconductor memory. Alternatively, the program may be provided as a data signal via communication networks.

Example

[1] Construction of predictive model

A model to predict the L3-L5 nerve root region was constructed using diffusion-weighted images of axial cross-sections in a range encompassing the L3-L5 nerves as input.

(1) Software and hardware used

MRICronGL: www.nitrc.org/projects/mricrogl; free software
Diffusion Toolkit: trackvis.org/download/; free software
med2image: github.com/FNNDSC/med2image; open-source Python package milesial/Pytorch-UNet: github.com/-milesial/Pytorch-UNet

(2) Creation of training data

DTI data from a total of 90 patients who underwent MRI imaging at the Department of Radiology, Chiba University Hospital was studied.

MRI data written to a DVD within the hospital was read into a PC in the DICOM format, and then converted to a file in the Nifti format using the dcm2niix tool included in MRICronGL.

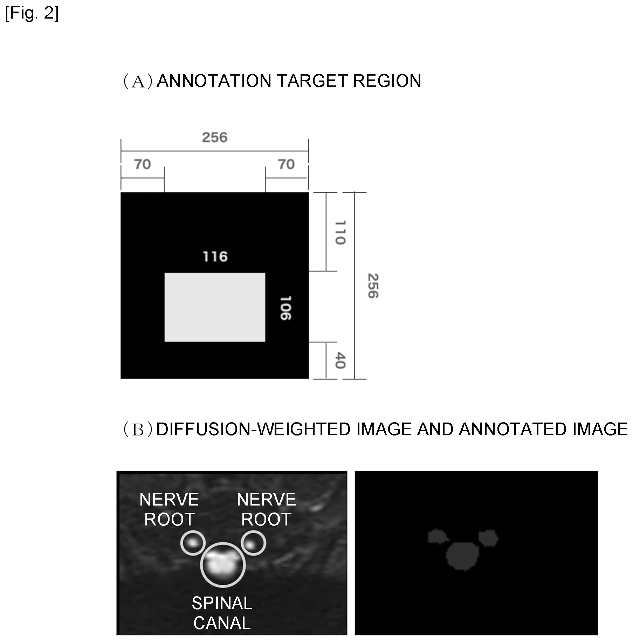

Next, the Nifti-format file obtained by the conversion and containing the DTI data and a Gradient Table file were input to the free software Diffusion Toolkit to reconstruct the data. Using med2image on the obtained Nifti-format file containing diffusion-weighted data, all slice images of the axial cross-sections were generated from diffusion-weighted data as jpg images with a size of $256 \times 256$ pixels. For all generated images, areas outside a region of $116 \times 106$ pixels region shown in Fig. 2(A) were filled with black, and the obtained diffusion-weighted images of the axial cross-sections were visually inspected one by one. For images showing the presence of nerve roots, annotations were made at two nerve root regions and the spinal canal region (Fig. 2(B)). Since the target nerve root regions are minute, this was done to improve learning accuracy by reducing unnecessary regions and increasing feature amounts.

The operation described above was performed on DTI data for the 90 subjects, yielding a total of 839 diffusion-weighted images and corresponding annotated images for two nerve root regions and the spinal canal region. The breakdown of the 839 images is as follows: patient data for approximately 90 individuals was acquired at three locations, namely, L3, L4, and L5, and in three portions, namely, proximal, mid, and distal, at each location.

(3) Construction of trained model

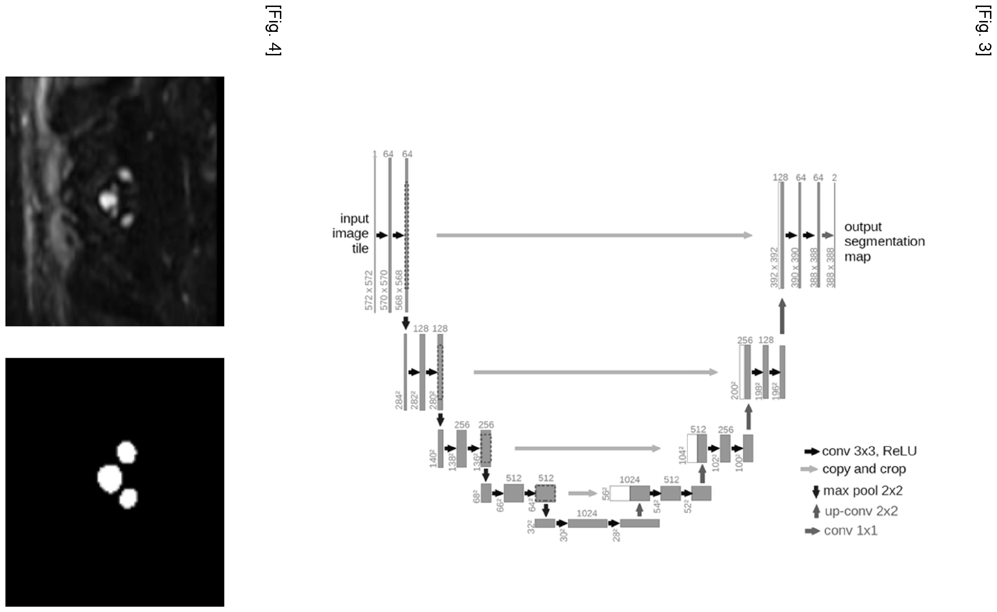

U-Net was used as a segmentation model. U-Net is a network for estimating segmentation of images and utilizes Fully Convolution Network, Deconvolution, and Skip-Connection. Fig. 3 shows a structure of U-Net. The structure of U-Net is an hourglass-shaped encoder-decoder network that forms a feature pyramid.

In addition, milesial/Pytorch-UNet was used as a segmentation model. Pytorch-UNet, which uses Python 3.6.0 and Pytorch 1.12.1, is a custom model based on U-Net that was trained from scratch on 5,000 images and achieved a Dice coefficient of 0.98 on over 100,000 test images.

The development and experimental environments are as follows.

OS: Windows 11
GPU: NVIDIA GeForce RTX 3070Ti
CUDA: 11.3
CuDNN: 8.5.0
Learning rate: 0.00001
Number of epochs: 100
Batch size: 1

Optimizer: RMSProp
Loss function: CrossEntropyLoss

(4) Evaluation of trained model

The F-score (also known as the Dice coefficient) was used as an evaluation index for the model. Table 1 shows a confusion matrix.

[Table 1]

|  | Positive | Negative |
|---|---|---|
| **True** | TP | TN |
| **False** | FP | FN |

A confusion matrix is a table used to show prediction accuracy in binary classification. A recall obtained therefrom is defined by Equation (1), a precision by Equation (2), and a harmonic mean of recall and precision (F-score) by Equation (3).

[Expression 3]

$$Recall = \frac{TP}{TP + FP} \qquad (1)$$

$$Precision = \frac{TP}{TP + FN} \qquad (2)$$

$$F = \frac{2}{\frac{1}{Precision} + \frac{1}{Recall}} = \frac{TP}{TP + \frac{1}{2}(FP + FN)} \qquad (3)$$

In a confusion matrix, recall represents a proportion of data that is predicted to be true to data that is actually true, and precision represents a proportion of data that is actually true to data that is predicted to be true. In other words, in prediction of data, a higher recall means fewer correct answers are missed while a higher precision means fewer incorrect answers are made, and a higher F-score being a harmonic mean of these two indices indicates a higher similarity between the predicted data and the original data.

The calculation of F-scores was performed using Pytorch.

Table 2 shows the highest-accuracy results among the F-scores and train loss values obtained by training a total of 839 pairs of diffusion-weighted images and annotated images across five training runs.

[Table 2]

| Item | Result |
|---|---|
| F-score | 0.77 |
| Train Loss | 0.15 |

Here, the calculated F-scores and train loss values are means of values calculated in each epoch.

In addition, Fig. 4 shows an original image and an actually obtained predicted image. A left diagram in Fig. 4 is the original image and a right diagram in Fig. 4 is the actually obtained predicted image.

Although the predicted image shows a larger region, it can be said that a total of three regions including two nerve root regions and one spinal canal region have been predicted.

(5) Preliminary review of annotation conditions

Annotation conditions in the training data have been reviewed in advance.

(i) In training data, a case where annotations were made at a total of three regions including two nerve root regions and one spinal canal region was compared with a case where annotations were made only at the two nerve root regions. As a result, the prediction accuracy of the nerve root regions improved in the former case. This is conceivably due to the model capturing a pattern of relative positions between the spinal canal region and the nerve root regions and using the captured pattern as additional contextual information for training.

(ii) In training data, a case where an annotation target region is set to $256 \times 256$ pixels was compared with a case where the annotation target region is set to $116 \times 106$ pixels.

As a result, a field of view (FOV) could be narrowed around the spinal cord, making the nerve root regions more clearly defined.

[1-1] Improvement of trained model

In "(2) Creation of training data" in [1] above, separate labels were used for annotation of the two nerve root regions and the spinal canal region (dural canal). The operation was performed on DTI data for the 90 subjects in a similar manner to [1] above, yielding a total of 839 diffusion-weighted images and corresponding annotated images for two nerve root regions and the spinal canal region.

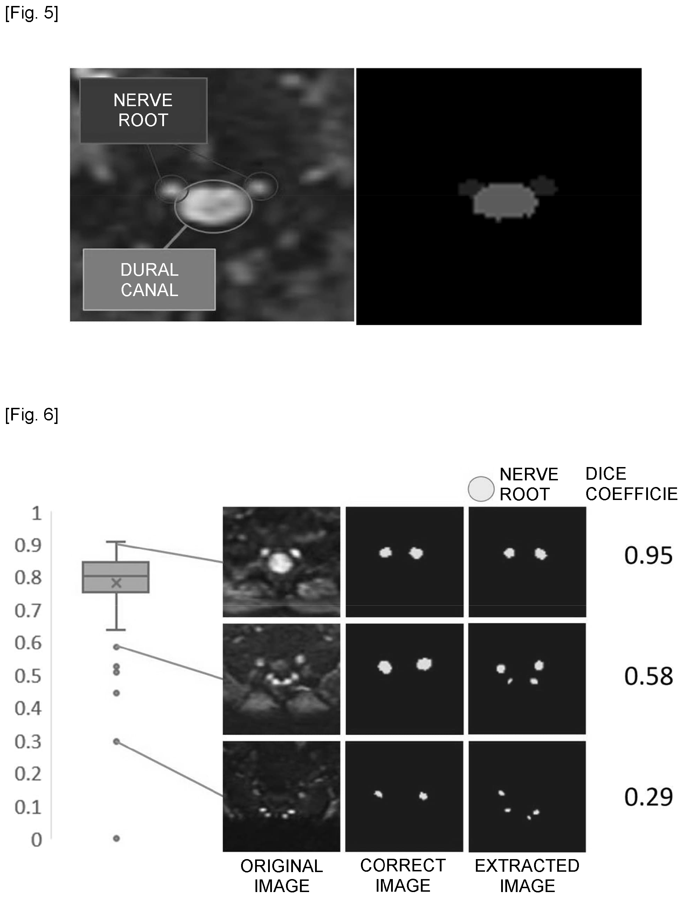

Fig. 5 shows a diffusion-weighted image and an annotated image.

In a similar manner to "(3) Construction of trained model" in [1] above, the model was trained by inputting diffusion-weighted images as input data and annotated images as training data into the network, thereby having the model learn how to label the input data. The pairs of diffusion-weighted images and annotated images for the 90 input cases were divided into Training: Validation: Test at a ratio of 50:25:15 (cases).

The learning rate that yielded the highest accuracy was $10^{-4}$, and ResNet34 was used as a feature extraction model.

In a similar manner to "(4) Evaluation of trained model" in [1] above, a segmentation task was evaluation using the Dice coefficient as an index. The Dice coefficient averaged $0.780 \pm 0.105$ on the test data.

Fig. 6 shows a result and extracted images.

The development environment and parameters are as follows.

OS: Windows 10
GPU: Tesla T4
Python: 3.8.16
Pytorch: 1.12.1
Segmentation models pytorch: 0.2.1
Learning rate: $10^{-2}$, $10^{-3}$, $10^{-4}$
Number of learning sessions: 40
Batch size: 6
Classification classes: 3 (background, spinal canal, nerve root)

[2] Automated measurement of FA value

An FA value was measured in an automated manner using the trained model constructed in [1] above and compared with an FA value obtained by manual measurement.

(1) Software used and development environment

The software used was the same as [1] above.

The development environment is as follows.

OS: MacOS Big Sur
Processor: 3.1GHz Intel Core i5
Memory: 16GB 1867 MHz DDR3

(2) Data used

From the DTI data from a total of 90 patients who underwent MRI imaging at the Department of Radiology, Chiba University Hospital used in constructing the predictive model described above, data of one patient was extracted and used.

(3) Generation of diffusion-weighted images

The MRI data was loaded into the PC in the DICOM format and converted to Nifti-format files using dcm2niix included in MRICronGL.

Next, the Nifti-format file obtained by the conversion and containing the DTI data and a Gradient Table file were input to Diffusion Toolkit to reconstruct the data. Parameters of Diffusion Toolkit were as follows.

Imaging model: DTI
Maximum b value: 1000
Gradient table: gradient_table
Orientation patch: Invert Y

Using med2image on the obtained Nifti-format file containing diffusion-weighted data, all slice images of the axial cross-sections were generated from diffusion-weighted data as jpg images with a size of $256 \times 256$ pixels.

(4-1) Manual measurement of FA value

Diffusion-weighted images of the axial cross-sections were visually inspected one by one, and for images in which the presence of nerve roots was observed, two nerve root regions were identified and designated as regions of interest (ROIs). All coordinate information and FA values of the ROIs were acquired and a mean FA value was calculated.

(4-2) Automated measurement of FA value

Using the trained model constructed in [1] above, the generated diffusion-weighted images of the axial cross-sections were read one by one, and two nerve root regions and one spinal canal region were extracted and generated as jpg.

The generated predicted images were binarized and subjected to image processing that removes a region with the largest area in the images to remove only the spinal canal region.

Using all images after undergoing image processing, all coordinate information and FA values of the predicted portions were acquired and a mean FA value was calculated.

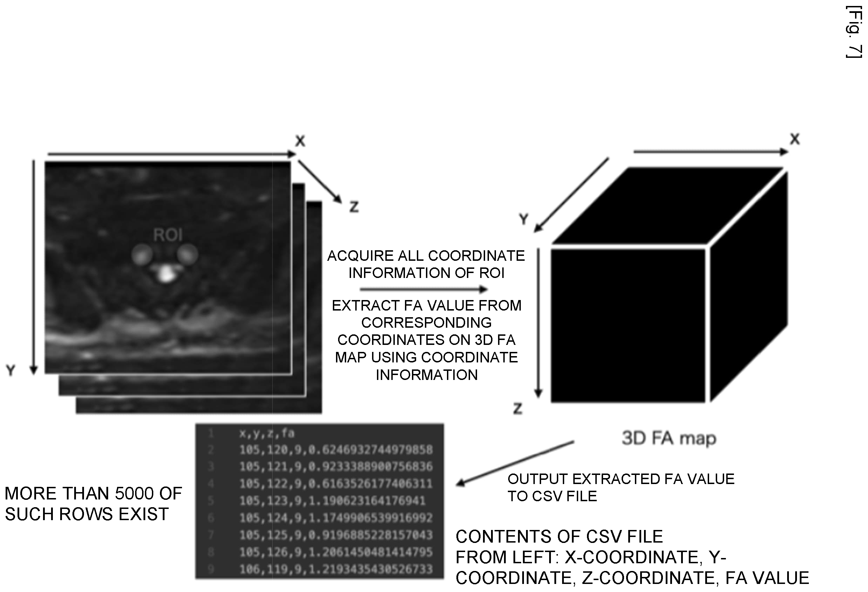

Fig. 7 schematically shows a method for measuring FA values in an automated manner.

(5) Result

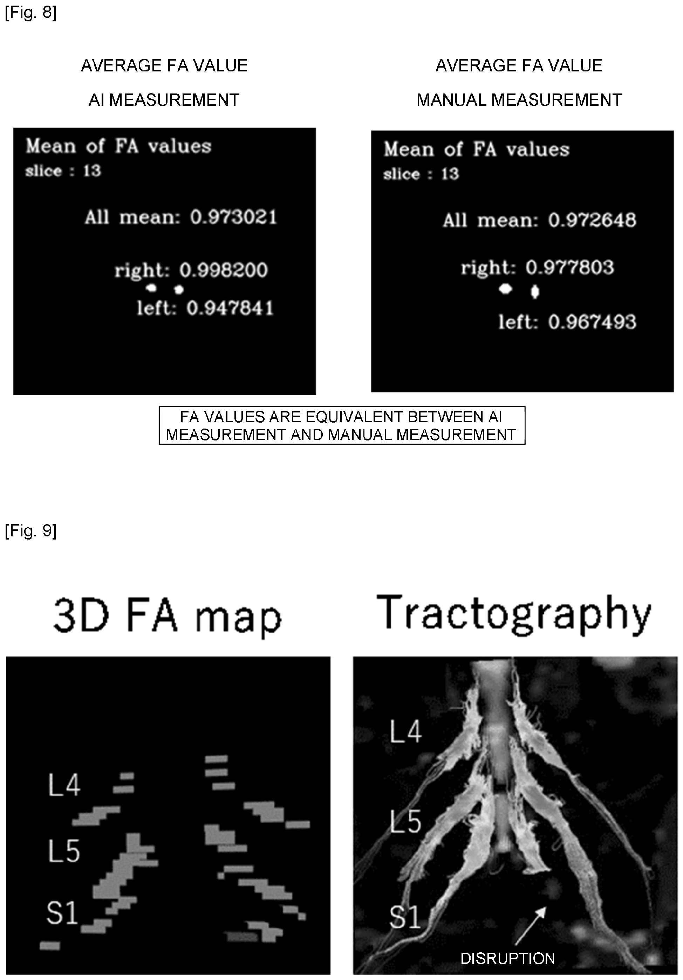

Fig. 8 shows a comparison result between automated measurement (AI measurement) of FA values and manual measurement of FA values. It was confirmed that the FA values obtained through automated measurement (AI measurement) and manual measurement are equivalent.

[3] Creation of lumbar nerve tractography

(1) Software used and development environment

The following software was used in addition to those used in [1] above.
Trackvis: trackvis.org/download/; free software

The development environment is as follows.

OS: MacOS Big Sur
Processor: 3.1GHz Intel Core i5
Memory: 16GB 1867 MHz DDR3

(2) Data used

From the DTI data from a total of 90 patients who underwent MRI imaging at the Department of Radiology, Chiba University Hospital used in constructing the predictive model described above, data of one patient was extracted and used.

(3) Generation of diffusion-weighted images

The MRI data was loaded into the PC in the DICOM format and converted to Nifti-format files using dem2niix included in MRICronGL.

Next, the Nifti-format file obtained by the conversion and containing the DTI data and a Gradient Table file were input to Diffusion Toolkit to reconstruct the data. Parameters of Diffusion Toolkit were as follows.

Imaging model: DTI
Maximum b value: 1000
Gradient table: gradient_table
Orientation patch: Invert Y

Using med2image on the obtained Nifti-format file containing diffusion-weighted data, all slice images of the axial cross-sections were generated from diffusion-weighted data as jpg images with a size of 256 × 256 pixels.

(4) Automated creation of lumbar nerve tractography

Using the trained model constructed in [1] above, the generated diffusion-weighted images of the axial cross-sections were read one by one, and two nerve root regions and one spinal canal region were extracted and generated as jpg.

The generated predicted images were binarized and subjected to image processing that removes a region with the largest area in the images to remove only the spinal canal region.

Based on the coordinate information of the nerve root regions remaining in the image, an ROI file for use with Trackvis was created in the Nifti format. Finally, the ROI file created with the Trackvis command was read to create the tractography of the lumbar nerve. The processing described above was implemented using a Python script.

(5) Result

Table 3 shows an average time required for creating lumbar nerve tractography in an automated manner three times, and an average time required for manually creating lumbar nerve tractography three times as a control.

The difference in the average time required between manually creating lumbar nerve tractography and creating lumbar nerve tractography in an automated manner is 160 seconds, demonstrating that automation enables creation in significantly less time than manual methods.

[Table 3]

| Manual (seconds) | Automated (seconds) |
| --- | --- |
| 351±4.3 | 191±3.0 |

[4] Creation of 3D FA map

(1) Data used

DTI data from 20 patients who underwent MRI imaging at the Department of Radiology, Chiba University Hospital and for whom tractography was available was studied.

(2) Creation of 3D FA map

Position coordinate information of nerve roots was extracted using the trained model constructed in [1] above.

In addition, FA values of corresponding positions were calculated. Specifically, NumPy arrays containing the respective FA values and ROI information were obtained using the following function within the Python library nibabel.
Function:

```
def get_nii_fdata(nii_path):

    nii = nib.load(nii_path)
    return nii.get_ fdata()
```

A slice image containing only pixel values within the ROI of each axial image was created and saved.

Two contours of a largest shape in the generated image were acquired, with a first contour stored as a left side and a second as a right side. The cv2.findContours function from OpenCV was used for the image processing.

Furthermore, rectangles circumscribing the obtained contours were drawn and coordinates of the upper-left corner and vertical and horizontal lengths of the respective rectangles enclosing the left and right sides were recorded. The cv2.boundingRect() function was used when drawing the rectangles.

To compare the FA values between the left and right sides, the average FA values for each side corresponding to the obtained ROI position were calculated and compared. When the left FA value was lower than the right FA value by 0.1 or more, left_flag = 1 was set, and when the right FA value was lower than the left FA value by 0.1 or more, right_flag = 1 was set. If there was no difference, both flags were set to 0. The coordinate information was saved in a csv file in a format of [left_or_right,x,y,z,fa,flag].

Next, based on the coordinates and the FA values obtained from the csv file, reconstruction was performed using Three.js. Three.js is a JavaScript library that generates objects from coordinate information.

First, a zero-padded array with a size of $40 \times 40 \times 40$ was created in which 1 was stored only at locations corresponding to entries in the csv file. In addition, 2 was stored in locations where flag = 1. Next, an object was generated by designating that locations of which the element is 1 are to be colored gray and locations of which the element is 2 are to be colored red.

Fig. 9 shows an example where locations of a disruption match between tractography and a 3D FA map.

## Claims

1. A medical image processing device comprising: a diffusion tensor imaging MRI data acquisition unit that acquires diffusion tensor imaging MRI data obtained by scanning the lumbar region of a patient with low back and leg pain;

   a coordinate information estimation unit that estimates coordinate information of a nerve root in a designated axial cross-section; and
   a measurement unit that measures an FA value of a nerve root of which coordinate information was estimated, wherein
   the diffusion tensor imaging MRI data includes diffusion-weighted image information and FA map information containing scalar information, vector information, and coordinate information,
   the coordinate information estimation unit estimates coordinate information of the nerve root in the designated axial cross-section using a pre-trained model trained using, as training data, annotated images in which annotations were added to two nerve root regions and a spinal canal region included in a same axial cross-sectional image among MRI diffusion-weighted images of the lumbar region, and
   the measurement unit measures, based on coordinate information of the nerve root estimated by the coordinate information estimation unit, an FA value of the nerve root of which coordinate information was estimated based on the scalar information and the coordinate information.

2. The medical image processing device according to claim 1, wherein the same axial cross-sectional image among the MRI diffusion-weighted images of the lumbar region is a cross-sectional image of at least one of the third lumbar vertebra (L3), the fourth lumbar vertebra (L4), and the fifth lumbar vertebra (L5).

3. The medical image processing device according to claim 2, wherein the same axial cross-sectional image among the MRI diffusion-weighted images of the lumbar region is a cross-sectional image of the third lumbar vertebra (L3), the fourth lumbar vertebra (L4), and the fifth lumbar vertebra (L5).

4. The medical image processing device according to claim 1, wherein the same axial cross-sectional image among the MRI diffusion-weighted images of the lumbar region is a plurality of cross-sectional images centered on a central portion of a same nerve root.

5. The medical image processing device according to claim 4, wherein the same axial cross-sectional image among the MRI diffusion-weighted images of the lumbar region is a cross-sectional image of proximal, central, and distal portions of a same nerve root.

6. The medical image processing device according to claim 1, wherein in the training data, the annotation of the nerve root region differs from the annotation of the spinal canal region.

7. The medical image processing device according to claim 1, wherein the designated axial cross-section is a plurality of consecutive axial cross-sections in the lumbar region of the patient, and the medical image processing device further comprises:
   a tractography generation unit that associates coordinate information of the nerve root in each axial cross-section estimated by the coordinate information estimation unit with the scalar information, the vector information, and the coordinate information of the FA map information created from the diffusion tensor imaging MRI data and that generates diffusion tensor tractography indicating a tract of nerve fibers.

8. The medical image processing device according to claim 1, wherein the designated axial cross-section is a plurality of consecutive axial cross-sections in the lumbar region of the patient, and the medical image processing device further comprises:
a 3D FA map generation unit that associates coordinate information of the nerve root in each axial cross-section estimated by the coordinate information estimation unit with an FA value measured by the measurement unit, reconstructs the nerve root in a three-dimensional space, and generates a 3D FA map.

9. A method for acquiring information on a spinal nerve, comprising the steps of:

acquiring diffusion tensor imaging MRI data obtained by scanning the lumbar region of a patient with low back and leg pain;
estimating coordinate information of a nerve root in a designated axial cross-section; and
measuring an FA value of a nerve root of which coordinate information was estimated, wherein
the diffusion tensor imaging MRI data includes diffusion-weighted image information and FA map information containing scalar information, vector information, and coordinate information,
the step of estimating the coordinate information involves estimating coordinate information of the nerve root in the designated axial cross-section using a pre-trained model trained using, as training data, annotated images in which annotations were added to two nerve root regions and a spinal canal region included in a same axial cross-sectional image among MRI diffusion-weighted images of the lumbar region, and
the step of measuring an FA value of the nerve root involves measuring, based on coordinate information of the nerve root estimated in the step of estimating coordinate information, an FA value of the nerve root of which coordinate information was estimated based on the scalar information and the coordinate information.

10. The method according to claim 9, wherein information on a spinal nerve acquired by the method according to claim 9 is an index for estimating the degree of spinal nerve disorder from a measured FA value.

11. A method for generating diffusion tensor tractography indicating a tract of nerve fibers of a spinal nerve, the method comprising the steps of:

acquiring diffusion tensor imaging MRI data obtained by scanning the lumbar region of a patient with low back and leg pain;
estimating coordinate information of a nerve root in each of a plurality of consecutive axial cross-sections in a lumbar region of a designated patient;
associating the information; and
generating diffusion tensor tractography indicating a tract of nerve fibers by superimposing each axial cross-section to three-dimensionally construct the axial cross-sections, wherein
the diffusion tensor imaging MRI data includes diffusion-weighted image information and FA map information containing scalar information, vector information, and coordinate information,
the step of estimating the coordinate information involves estimating coordinate information of the nerve root in the designated axial cross-section using a pre-trained model trained using, as training data, annotated images in which annotations were added to two nerve root regions and a spinal canal region included in a same axial cross-sectional image among MRI diffusion-weighted images of the lumbar region, and
the step of associating the information involves associating coordinate information of the nerve root in each axial cross-section estimated in the step of estimating the coordinate information of the nerve root with the scalar information, the vector information, and the coordinate information of the FA value map created from the diffusion tensor imaging MRI data.

12. A method for generating a 3D FA map, comprising the steps of:

acquiring diffusion tensor imaging MRI data obtained by scanning the lumbar region of a patient with low back and leg pain;
estimating coordinate information of a nerve root in each of a plurality of consecutive axial cross-sections in a lumbar region of a designated patient;
measuring an FA value of a nerve root of which coordinate information was estimated; and

reconstructing the nerve root in a three-dimensional space using coordinate information of the nerve root of which coordinate information was estimated and the measured FA value corresponding to the coordinate information, wherein

the diffusion tensor imaging MRI data includes diffusion-weighted image information and FA map information containing scalar information, vector information, and coordinate information,

the step of estimating the coordinate information involves estimating coordinate information of the nerve root in the designated axial cross-section using a pre-trained model trained using, as training data, annotated images in which annotations were added to two nerve root regions and a spinal canal region included in a same axial cross-sectional image among MRI diffusion-weighted images of the lumbar region, and

the step of measuring an FA value of the nerve root involves measuring, based on coordinate information of the nerve root estimated in the step of estimating coordinate information, an FA value of the nerve root of which coordinate information was estimated based on the scalar information and the coordinate information.

13. The method for generating a 3D FA map according to claim 12, further comprising the steps of:

comparing measured FA values of two nerve roots included in a same axial cross-sectional image and of which coordinate information was estimated; and

distinguishably displaying a region of nerve roots where a predetermined difference exists between the measured FA values of the two nerve roots included in a same axial cross-sectional image and of which coordinate information was estimated from a region of other nerve roots.

14. A program for causing a computer to execute each step of the method for acquiring information on a spinal nerve according to claim 9, the method for generating diffusion tensor tractography indicating tracts of nerve fibers of a spinal nerve according to claim 11, or the method for generating a 3D FA map according to claim 12.

[Fig. 1]

E P 4 772 112 A1

[Fig. 2]

（A）ANNOTATION TARGET REGION

（B）DIFFUSION-WEIGHTED IMAGE AND ANNOTATED IMAGE

[Fig. 3]

[Fig. 4]

[Fig. 5]

[Fig. 6]

[Fig. 7]

3D FA map

OUTPUT EXTRACTED FA VALUE
TO CSV FILE

CONTENTS OF CSV FILE
FROM LEFT: X-COORDINATE, Y-
COORDINATE, Z-COORDINATE, FA VALUE

ACQUIRE ALL COORDINATE
INFORMATION OF ROI

EXTRACT FA VALUE FROM
CORRESPONDING
COORDINATES ON 3D FA
MAP USING COORDINATE
INFORMATION

MORE THAN 5000 OF
SUCH ROWS EXIST

x,y,z,fa
105,120,9,0.6246932744979858
105,121,9,0.9233388900756836
105,122,9,0.6163526177406311
105,123,9,1.1906231641769411
105,124,9,1.1749906539916992
105,125,9,0.9196885228157043
105,126,9,1.2061450481414795
106,119,9,1.2193434305426733

[Fig. 8]

AVERAGE FA VALUE

AI MEASUREMENT

AVERAGE FA VALUE

MANUAL MEASUREMENT

FA VALUES ARE EQUIVALENT BETWEEN AI
MEASUREMENT AND MANUAL MEASUREMENT

[Fig. 9]

## INTERNATIONAL SEARCH REPORT

| International application No. |
| --- |
| **PCT/JP2024/031370** |

**A. CLASSIFICATION OF SUBJECT MATTER**

***A61B 5/055***(2006.01)i

FI: A61B5/055 380; A61B5/055 382

According to International Patent Classification (IPC) or to both national classification and IPC

**B. FIELDS SEARCHED**

Minimum documentation searched (classification system followed by classification symbols)

A61B5/055; G01N24/00-24/14; G01R33/20-33/64; G06T7/00-7/90

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | SAKAI, T. et al., Functional Assessment of Lumbar Nerve Roots Using Coronal-plane Single-shot Turbo Spin-echo Diffusion Tensor Imaging, Magnetic Resonance in Medical Sciences. 13 June 2019, vol. 19, no. 2, pp. 159-165, DOI: 10.2463/mrms.tn.2019-0014 <br> entire text, all drawings | 1-14 |
| A | DAULEAC, C. et al., Full cervical cord tractography: A new method for clinical use, Frontiers in Neuroanatomy, 27 September 2022, vol. 16, pp. 1-10, DOI: 10.3389/fnana.2022.993464 <br> entire text, all drawings | 1-14 |
| A | EGUCHI, Y. et al., Quantitative Evaluation and Visualization of Lumbar Foraminal Nerve Root Entrapment by Using Diffusion Tensor Imaging: Preliminary Results, American Journal of Neuroradiology, 01 November 2011, vol. 32, issue 10, pp. 1824-1829, DOI: 10.3174/ajnr.A2681 <br> entire text, all drawings | 1-14 |

[✓] Further documents are listed in the continuation of Box C.    [✓] See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **01 November 2024** | **12 November 2024** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**EP 4 772 112 A1**

## INTERNATIONAL SEARCH REPORT

| | International application No. |
|---|---|
| | **PCT/JP2024/031370** |

**C. DOCUMENTS CONSIDERED TO BE RELEVANT**

| Category* | Citation of document, with indication, where appropriate, of the relevant passages | Relevant to claim No. |
|---|---|---|
| A | FAN, G. et al., Deep Learning-Based Automatic Segmentation of Lumbosacral Nerves on CT for Spinal Intervention: A Translational Study, American Journal of Neuroradiology, 01 June 2019, vol. 40, issue 6, pp. 1074-1081, DOI: 10.3174/ajnr.A6070 entire text, all drawings | 1-14 |
| A | US 2020/0261051 A1 (MEDOS INTERNATIONAL SARL) 20 August 2020 (2020-08-20) entire text, all drawings | 1-14 |
| A | CN 114723730 A (SOUTHERN MEDICAL UNIVERSITY) 08 July 2022 (2022-07-08) entire text, all drawings | 1-14 |

Form PCT/ISA/210 (second sheet) (July 2022)

**INTERNATIONAL SEARCH REPORT**
Information on patent family members

International application No.

**PCT/JP2024/031370**

| Patent document cited in search report | | | Publication date (day/month/year) | Patent family member(s) | | | Publication date (day/month/year) |
|---|---|---|---|---|---|---|---|
| US | 2020/0261051 | A1 | 20 August 2020 | WO | 2020/165858 | A1 | |
| CN | 114723730 | A | 08 July 2022 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **EGUCHI Y** ; **OHTORI S** ; **ORITA S et al.** Preliminary Results. *American Journal of Neuroradiology*, 2011, vol. 32, 1824-1829

- **EGUCHI Y** ; **OHTORI S** ; **YAMASHITA M et al.** *European Spine Journal*, 2010, vol. 19, 1874-1882
- **EGUCHI Y** ; **OHTORI S** ; **YAMASHITA M et al.** *Neuroradiology*, 2011, vol. 53, 633-641